# EUROPEAN PATENT SPECIFICATION

(11) **EP 3 942 298 B1**
(45) Date of publication and mention of the grant of the patent: **07.05.2025**
(21) Application number: 20774343.6
(22) Date of filing: 20.03.2020
(51) Int. Cl.: G01N 33/53, G01N 33/68

(54) **METHOD FOR INCREASING CELL SUSCEPTIBILITY TO COMPLEMENT-MEDIATED LYSIS**
VERFAHREN ZUR ERHÖHUNG DER ZELLANFÄLLIGKEIT FÜR KOMPLEMENTVERMITTELTE LYSE
MÉTHODE D'AUGMENTATION DE LA SENSIBILITÉ CELLULAIRE À LA LYSE À MÉDIATION PAR LE COMPLÉMENT

(30) Priority: 21.03.2019 US 201962821641 P
(43) Date of publication of application: 26.01.2022
(73) Proprietor: The UAB Research Foundation, Birmingham, AL 35233 (US)
(72) Inventor: TECTOR, Joe, Vestavia Hills, Alabama 35216 (US); TECTOR, Matt, Carmel, Indiana 46074 (US)
(74) Representative: Wilding, James Roger
(86) International application number: PCT/US2020/023747
(87) International publication number: WO 2020/191255

(56) References cited:
- WO-A1-2016/096788
- US-A1- 2014 099 733
- US-A1- 2018 044 686
- LADOWSKI JOSEPH M. ET AL: "Examining the Biosynthesis and Xenoantigenicity of Class II Swine Leukocyte Antigen Proteins", THE JOURNAL OF IMMUNOLOGY, vol. 200, no. 8, 15 April 2018 (2018-04-15), US, pages 2957 - 2964, XP055979631, ISSN: 0022-1767, Retrieved from the Internet <URL:https://www.ncbi.nlm.nih.gov/pmc/articles/PMC5978423/pdf/nihms954540.pdf> DOI: 10.4049/jimmunol.1800022
- ZHANG JUNFANG ET AL: "Potential Antigens Involved in Delayed Xenograft Rejection in a Ggta1/Cmah Dko Pig-to-Monkey Model", SCIENTIFIC REPORTS, vol. 7, no. 1, 1 December 2017 (2017-12-01), XP055979803, Retrieved from the Internet <URL:https://www.nature.com/articles/s41598-017-10805-0.pdf> DOI: 10.1038/s41598-017-10805-0
- GREGORY R. MARTENS ET AL: "Humoral Reactivity of Renal Transplant-Waitlisted Patients to Cells From GGTA1/CMAH/B4GalNT2, and SLA Class I Knockout Pigs :", TRANSPLANTATION, vol. 101, no. 4, 1 April 2017 (2017-04-01), GB, pages e86 - e92, XP055544939, ISSN: 0041-1337, DOI: 10.1097/TP.0000000000001646
- ASTRID J.F. THIELEN, IRIS M.VAN BAARSEN, MARLIEKE L.JONGSMA, SACHA ZEERLEDER, ROBBERT M.SPAAPEN, DIANA WOUTERS: "CRISPR/Cas9 generated human CD 46, CD 55 and CD 59 knockout cell lines as a tool for complement research", JOURNAL OF IMMUNOLOGICAL METHODS, vol. 456, 12 February 2018 (2018-02-12), pages 15 - 22, XP055742307
- DATABASE Nucleotide [online] NCBI; 10 August 2009 (2009-08-10), "Sus scrofa chromosome 9 clone CH242-216P17, WORKING DRAFT SEQUENCE, 5 unordered pieces", XP055742309, Database accession no. CU928329
- DATABASE UniProtKB [online] UniProt; 12 September 2018 (2018-09-12), "RecName: Full=Membrane cofactor protein {ECO:0000256|ARBA:ARBA00017517};", XP055742314, Database accession no. A0A2Y9TFC8

## Description

### SEQUENCE LISTING

This application contains a sequence listing filed in electronic form as an ASCll.txt file entitled "2221042940_ST25" created on March 18, 2020.

### BACKGROUND

Xenotransplantation (transplant of organs, tissues and cells from a donor of a different species) could effectively address the shortage of human donor pancreases. Xenotransplants are also advantageously (i) supplied on a predictable, non-emergency basis; (ii) produced in a controlled environment; and (iii) available for characterization and study prior to transplant.

Depending on the relationship between donor and recipient species, the xenotransplant can be described as concordant or discordant. Concordant species are phylogenetically closely related species (e.g., mouse to rat). Discordant species are not closely related (e.g., pig to human). Pigs have been the focus of most research in the xenotransplanation area, since the pig shares many anatomical and physiological characteristics with human. Pigs also have relatively short gestation periods, can be bred in pathogen-free environments and may not present the same ethical issues associated with animals not commonly used as food sources (e.g., primates). Scientific knowledge and expertise in the field of pig-to-primate xenotransplantation has grown rapidly over the last decade, resulting in the considerably prolonged survival of primate recipients of lifesaving porcine xenografts. (Cozzi et al., Xenotransplantation 16: 203-214. 2009).

Xenograft rejection can be divided into three phases: hyperacute rejection, acute humoral xenograft rejection, and T cell-mediated cellular rejection. Hyperacute rejection (HAR) is a very rapid event that results in irreversible graft damage and loss within minutes to hours following graft reperfusion. It is triggered by the presence of xenoreactive natural antibodies present within the recipient at the time of transplantation. Humans have a naturally-occurring antibody to the α1,3-galactose (Gal) epitope found on pig cells. This antibody is produced in high quantity and is the principle mediator of HAR. Genetic removal of the α1,3-galactose (Gal) epitope from pig cells as αGT (GTKO) results in GTKO pigs that do not undergo HAR. Ladowski et al., Journal of Immunology (2018);200(8):2957-2964 describes examining the biosynthesis and xenoantigenicity of class II swine leukocyte antigen proteins. Zhang et al., Scientific Reports (2017);7:10024 relates to potential antigens involved in delayed xenograft rejection in a Ggta1/Cmah DKO pig-to-monkey model. Martens et al., Transplantation (2017);101(4):e86-e92 relates to humoral reactivity of renal transplant-waitlisted patients to cells from GGTA1/CMAH/B4GalNT2, and SLA class I knockout pigs.

### SUMMARY OF THE INVENTION

The invention provides a method of detecting transplant donor organ-reactive antibodies in a human patient, the method comprising the steps of: (a) reducing the expression of at least one complement inhibitor by a population of cells derived from a candidate animal transplant donor organ by genetically modifying the population of cells, wherein the transplant donor organ is an organ, tissue or cells from an animal for use as xenografts; (b) contacting the population of genetically-modified cells with a sample of serum isolated from a human patient desiring to receive the transplant donor organ; and (c) detecting lysis of the genetically modified population of cells, wherein lysis indicates if the patient desiring to receive the transplant donor organ expresses donor organ-reactive antibodies, and wherein the lysis is detectable or increased when compared with a population of cells derived from the organ and not genetically modified to have a reduction in at least one expressed complement inhibitor.

In some embodiments, the population of cells can be donor lymphocytes.

In some embodiments, the population of cells can be derived from a kidney.

In some embodiments, the population of cells can be renal endothelial cells.

In some embodiments, the method can further comprise: (i) obtaining a tissue sample from the organ; and (ii) generating a population of genetically-modified organ-derived cells comprising an inactive complement inhibitor encoding gene generated by transfecting the cells with at least one expression vector encoding a guide RNA and CRISPR Cas9, wherein the guide RNA is specific for a complement inhibitor.

In some embodiments, the inactive complement inhibitor encoding gene can be selected from the group consisting of CD46, CD55, and CD59.

In some embodiments, the complement inhibitor can be CD46.

In some embodiments, the guide RNA inactivating an inactive complement inhibitor encoding gene can comprise a nucleotide sequence selected from the group consisting of SEQ ID NO: 1, SEQ ID NO: 3; SEQ ID NO: 5, SEQ ID NO: 7, SEQ ID NO: 9, and SEQ ID NO: 11, and a nucleotide sequence complementary to a nucleotide sequence selected from the group consisting of SEQ ID NO: 2, SEQ ID NO: 4; SEQ ID NO: 6, SEQ ID NO: 8, SEQ ID NO: 10, and SEQ ID NO: 12.

In some embodiments, the complement inhibitor can be CD46-like and the guide RNA for CRISPR Cas9-based inactivation can hybridize under physiological conditions with the sequence of SEQ ID NO: 19, or a complement thereof, and can have a nucleotide sequence of SEQ ID NO: 20.

In some embodiments, the expression vector can be plasmid PX330 or PX458 and the expression plasmid expresses Cas9 when the plasmid is transfected into a mammalian cell.

In some embodiments, the expression vector can comprise a nucleotide sequence having at least 85% similarity to a nucleotide sequence selected from the group consisting of SEQ ID Nos.: 13-18.

Another aspect of the disclosure encompasses embodiments of a method of detecting transplant donor organ-reactive antibodies in a human patient, the method comprising the steps of: (a) reducing the expression of at least one complement inhibitor by a population of renal endothelial cells derived from a candidate animal transplant donor kidney by genetically modifying the population of the cells, wherein the genetic modification can be by the steps of: (i) obtaining a tissue sample from the kidney or lymphocytes; and (ii) generating a population of genetically-modified kidney-derived cells comprising an inactive CD46 complement inhibitor-encoding gene generated by transfecting the renal endothelial cells with at least one expression vector encoding a guide RNA and CRISPR Cas9, wherein the guide RNA is specific for the complement inhibitor CD46 and can comprises a nucleotide sequence selected from the group consisting of SEQ ID NO: 1 and SEQ ID NO: 7, and a nucleotide sequence complementary to a nucleotide sequence selected from the group consisting of SEQ ID NO: 2 and SEQ ID NO: 8, and wherein the expression vector can comprise a nucleotide sequence having at least 85% similarity to a nucleotide sequence selected from SEQ ID NO: 13 or 16; (b) contacting the population of genetically-modified renal endothelial cells with a sample of serum isolated from a human patient desiring to receive the transplant donor organ; and (c) detecting lysis of the genetically modified population of renal endothelial cells, wherein lysis indicates if the patient desiring to receive the transplant donor organ expresses donor organ-reactive antibodies, and wherein the lysis is detectable or increased when compared with a population of renal endothelial cells derived from the organ and not genetically modified to have a reduction in at least one expressed complement inhibitor.

Each genetically modified cell may be a mammalian cell, wherein the cell is derived from a candidate animal transplant donor organ tissue, and wherein the cell is genetically modified to have a reduced or no expression of a complement inhibitor.

In some embodiments, the complement inhibitor can be CD46, CD55, or CD59.

In some embodiments, the complement inhibitor can be CD46-like, CD46, CD55, or CD59.

In some embodiments, the complement inhibitor can be CD46-like and can have an amino acid sequence at least 90% similar to the amino acid sequence SEQ ID NO: 23.

In some embodiments, the complement inhibitor can be CD46-like and can be encoded by a nucleotide sequence having at least 90% similarity with the nucleotide sequence of SEQ ID NO: 19.

In some embodiments, the cell can be genetically modified by a deletion by CRISPR Cas9 of all or a fragment of the genome of the cell encoding the complement inhibitor.

In some embodiments, the cell can be a population of cultured cells.

In some embodiments, the donor tissue can be obtained from an organ selected from the group consisting of a kidney, a lung, a heart, muscle, a skin tissue, or lymphocytes.

In some embodiments, the candidate organ is selected from the group consisting of a kidney, a lung, a heart, muscle, and a skin tissue.

In some embodiments, the candidate organ is a kidney.

In some embodiments, the genetically modified mammalian cell is a renal endothelial cell or lymphocyte.

These and further embodiments are set out in the appended claims.

### BRIEF DESCRIPTION OF THE DRAWINGS

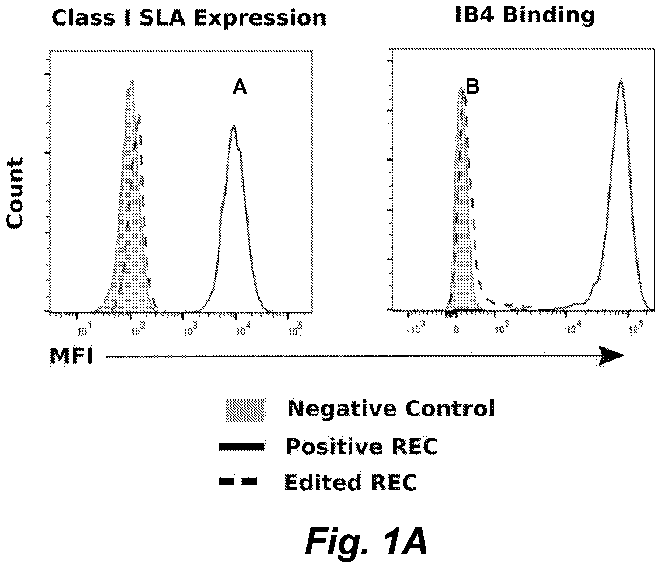

Figs. 1A-1C illustrate the creation of renal endothelial cells (RECs) deficient in CD46. Founder renal endothelial cells were made deficient in the GGTA1 gene and in class I SLA genes using CRISPR/Cas9. Consequently, class I SLA proteins and the alpha galactose carbohydrates were not expressed on the surface of these cells. The flow cytometric analyses of the founder cells are represented by dotted-line histograms. Negative control cells were unstained (gray histograms), positive control REC with intact class I SLA and GGTA1 genes are shown with a solid line histogram. These immortal REC were treated with CRISPR/Cas9 targeted to the CD46 gene.
Fig. 1A illustrates Class I proteins detected using a monoclonal antibody (Panel A) and IB4 lectin used to detect expression of the alpha gal epitope (Panel B).

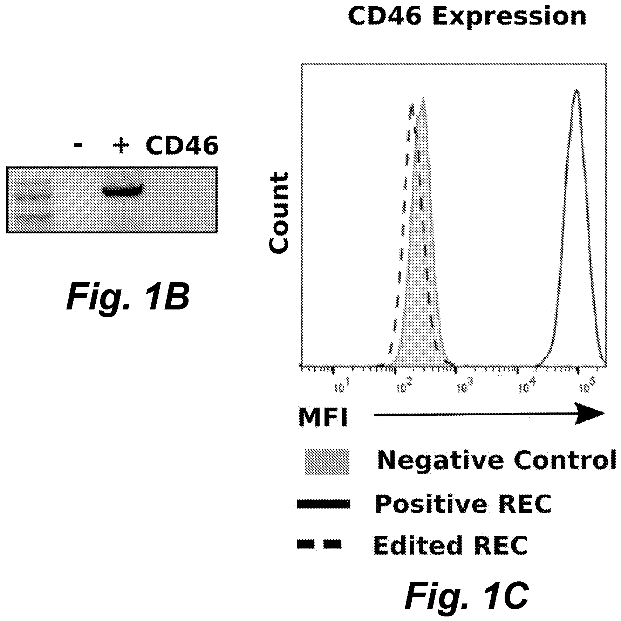

Fig. 1B shows a lack of PCR product (lane marked by a "-") indicating the CD46 gene had been disrupted. Cells were isolated and evaluated for transcription of the CD46 gene using reverse-transcriptase PCR. The parental REC, having an intact CD46 gene, were used as a positive control and demonstrated a clear band representing a CD46 gene product (lane marked by a "+").
Fig. 1C shows cell-surface CD46 expression evaluated in the parental cell (solid-line histogram), and in the CD46 knockout (dotted-line histogram). Unstained cells were used as a negative control (gray histogram).

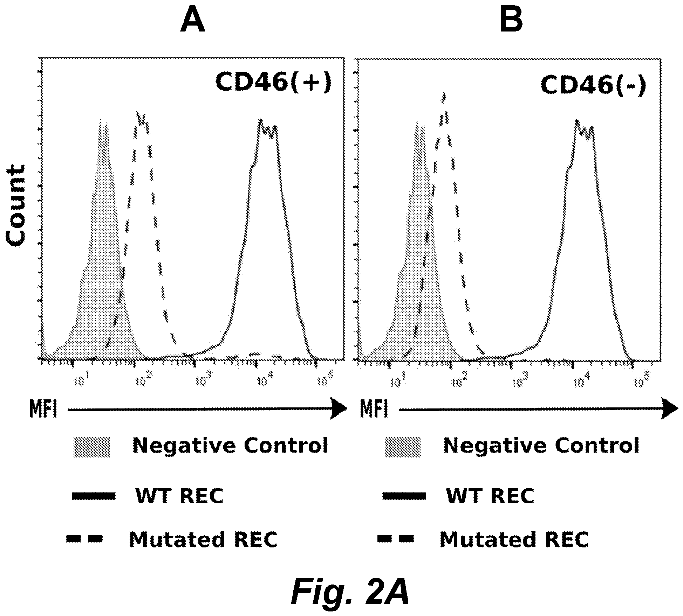

Fig. 2A illustrates the expression of two known exoantigens by CD46 KO REC. The parental REC had been treated with CRISPR/Cas9 that targeted the B4GalNT2 gene. Flow cytometry was performed using the DBA lectin to probe for N-acetyl-galactosamine, a xenoantigen produced by the B4GalNT2 enzyme. Both the parental (Panel A) and CD46-KO (Panel B) cells were tested. Results are displayed as dotted-line histograms. The same negative control (gray histograms-representing unstained cells) and the positive control cells with intact B4GalNT2 (solid-line histograms) were used in panels A and B.

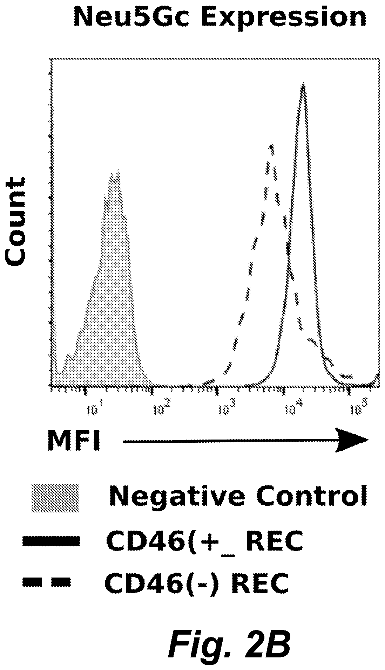

Fig. 2B illustrates the expression levels of the second xenoantigen, Neu5Gc, also evaluated using a Neu5Gc-specific monoclonal antibody. Unstained cells were used as a negative control (gray histogram). Expression of Neu5Gc by the parental cells is shown with a solid-line histogram while a dotted-line histogram represents the CD46-KO cells.

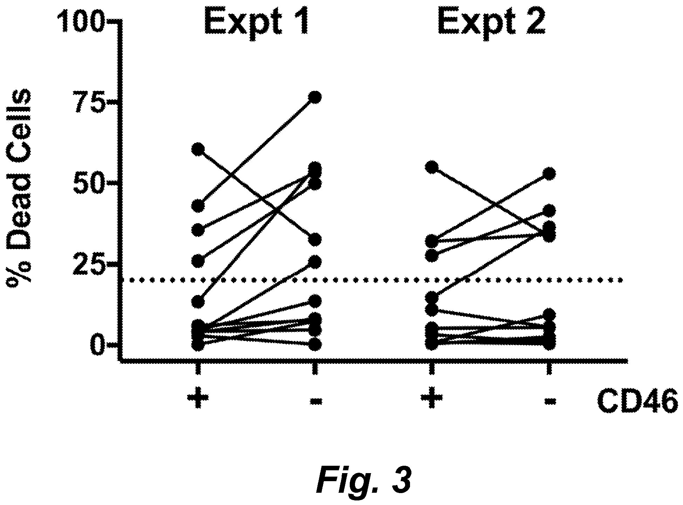

Fig. 3 illustrates complement-dependent cellular cytotoxicity analyses of CD46(+) parental REC *versus* CD46(-) RECs. A flow-cytometric based CDC assay was performed by incubating both types of REC with circulating antibodies collected from 13 different humans. The % dead cells were calculated by subtracting % death observed with baby rabbit complement alone versus death observed with human antibodies in addition to baby rabbit complement. Lines highlight the levels of death observed for each human antibody sample on both cell lines. The experiment was performed twice with all 13 samples.

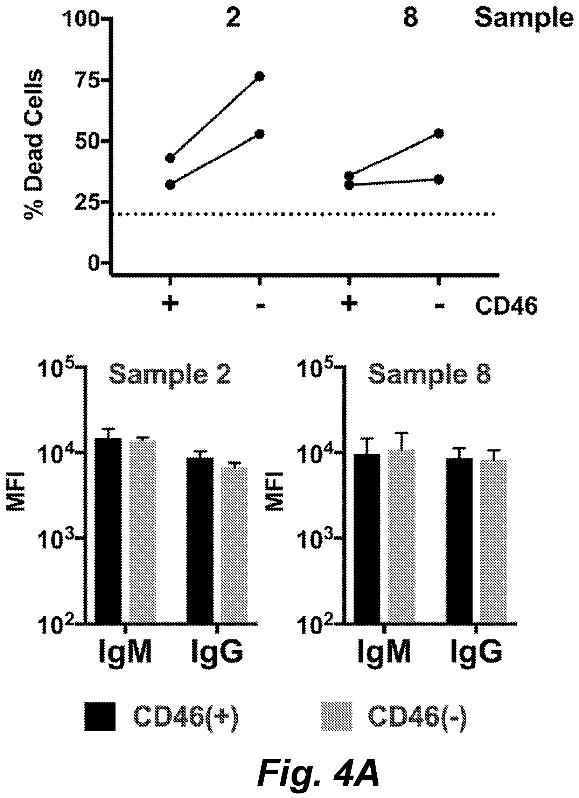

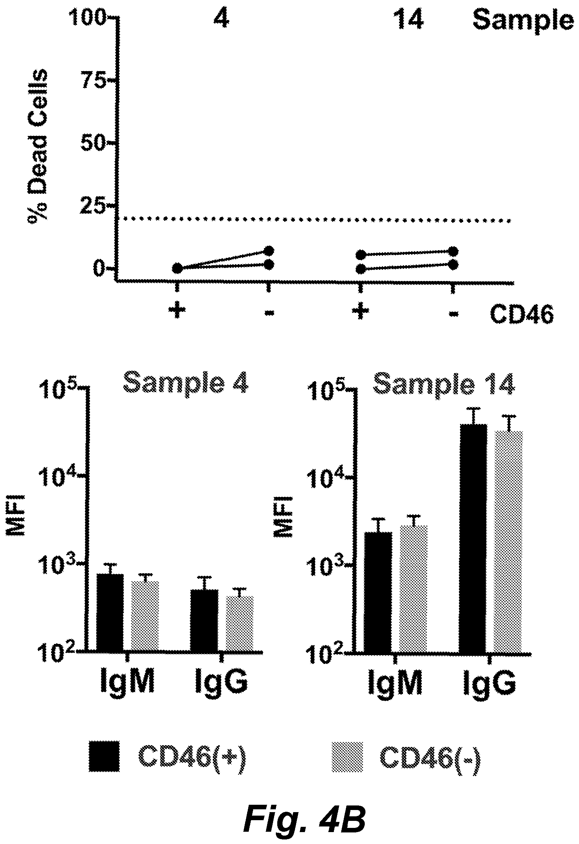

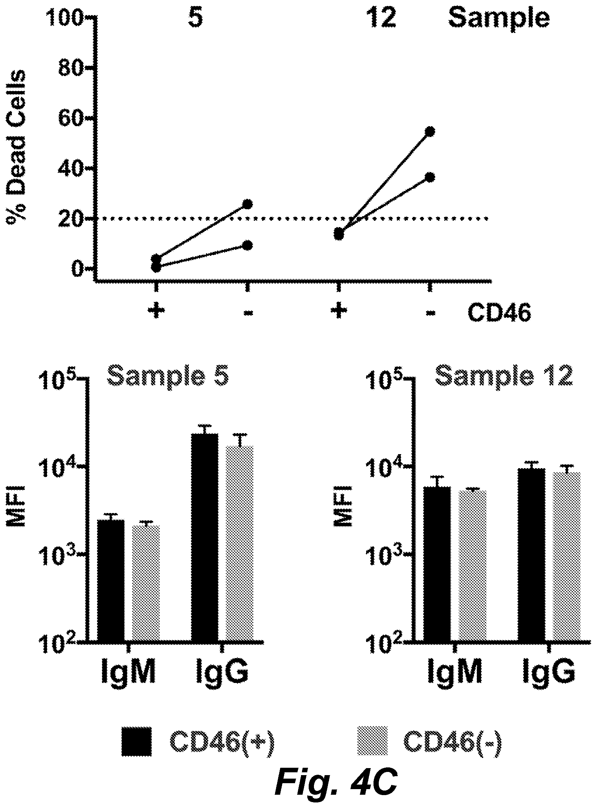

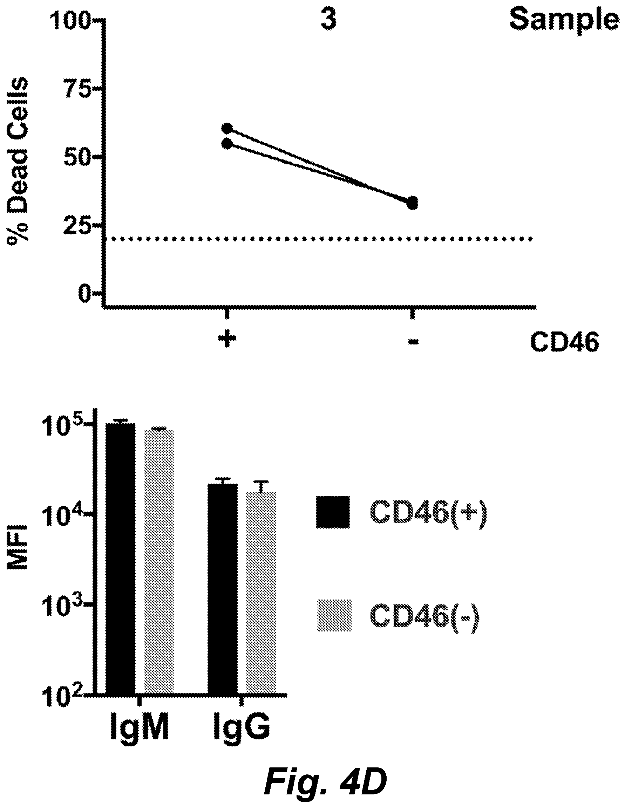

Figs. 4A-4D illustrate the comparison of cell lysis activity with IgM and IgG Binding Levels. The cells used in Fig. 3 were tested for levels of IgM and IgG binding against the 13 human samples. Three patterns of CDC results were used to categorize the various samples. Representative examples from the data shown in Fig. 3 are shown to highlight these categories.
Fig. 4A shows two examples of CDC that were positive (greater than 20% dead cells) regardless of the presence or absence of CD46.
Fig. 4B shows two examples of CDC that are negative (less than 20% dead cells) regardless of the presence or absence of CD46.
Fig. 4C shows CDC that are negative when using CD46(+) cells, but become positive on CD46(-) cells
Fig. 4D shows CDC that showed less lysis of CD46(-) cells than of CD46(+) cells. Flow-cytometric analyses of the antibody binding to these samples was performed in triplicate to examine relative IgG and IgM binding to CD46(+) and CD46(-) REC. The bar graphs below each CDC plot represent the results of the IgG and IgM analyses.

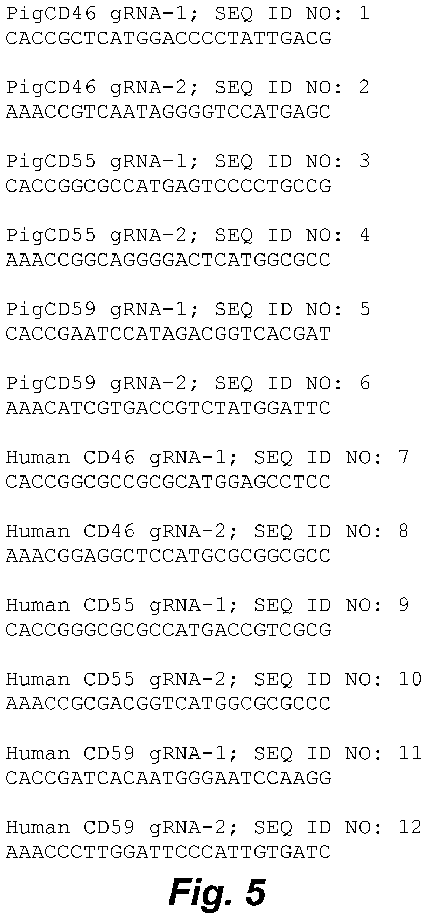

Fig. 5 illustrates the nucleotide sequences SEQ ID NO: 1-12.

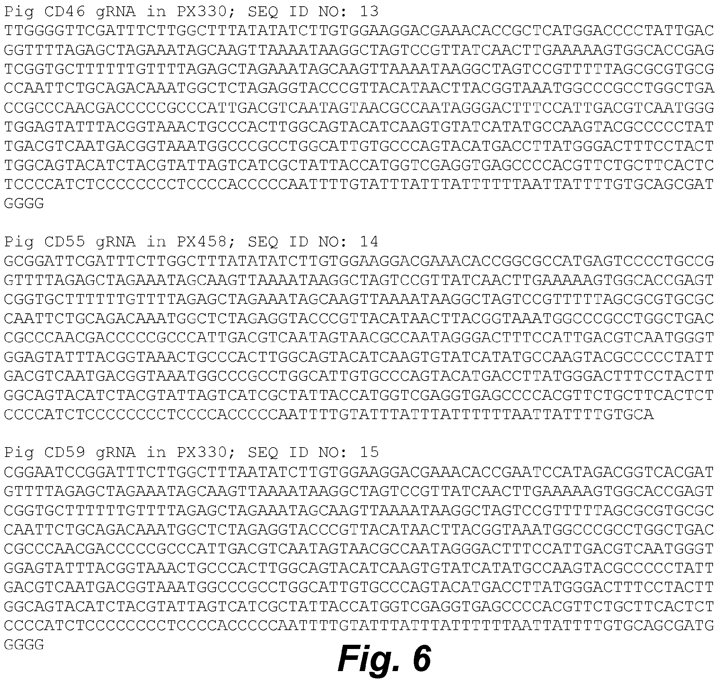

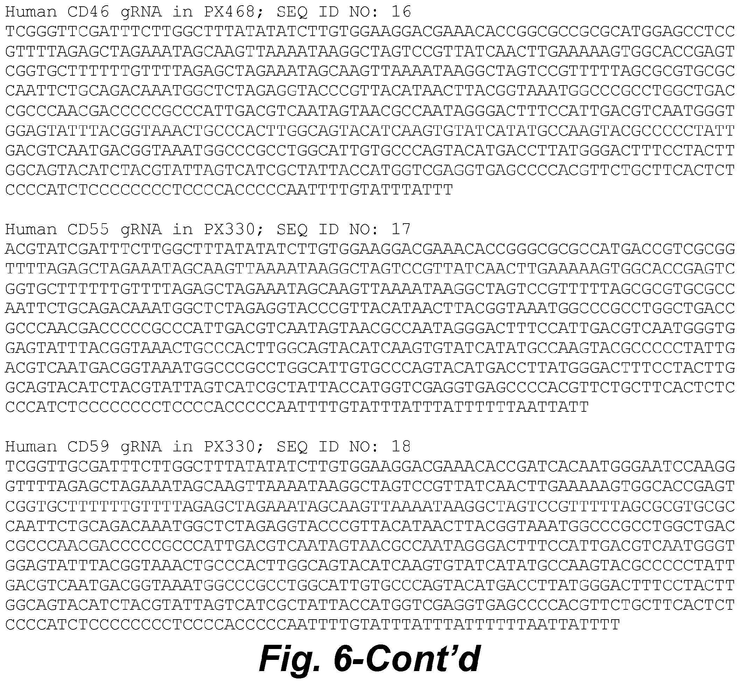

Fig. 6 illustrates the nucleotide sequences SEQ ID NO: 13-18.
Fig. 7 illustrates the nucleotide sequence (SEQ ID NO: 19) having the Accession Number XM_003482667.3 and being an mRNA nucleotide sequence encoding a porcine CD46-like complement inhibitor. The gRNA position is underlined. A guide RNS sequence (SEQ ID NO: 20 is also shown).
Fig. 8 illustrates an amino acid sequence alignment showing the similarities between the amino acid sequences human CD46 (SEQ ID NO: 21); porcine CD46 (SEQ ID NO: 22), and porcine CD46-like (SEQ ID NO: 23) complement inhibitors.

### DETAILED DESCRIPTION

The technical information set out below may in some respects go beyond the scope of the invention, which is defined exclusively by the appended claims. The additional technical information is provided to place the actual invention in a broader technical context and to illustrate possible related technical developments. In addition, incidental references to methods for treatment of the human or animal body by surgery or therapy and diagnostic methods practised on the human or animal body are not to be construed as claiming protection for such method as such but are instead to be construed as referring to products, in particular substances or compositions for use in any of these methods.

The terminology used herein serves the purpose of describing particular embodiments only, and is not intended to be limiting, since the scope of the present disclosure will be limited only by the appended claims.

Where a range of values is provided, each intervening value, to the tenth of the unit of the lower limit unless the context clearly dictates otherwise, between the upper and lower limit of that range and any other stated or intervening value in that stated range, is encompassed within the disclosure. The upper and lower limits of these smaller ranges may independently be included in the smaller ranges and are also encompassed within the disclosure, subject to any specifically excluded limit in the stated range. Where the stated range includes one or both of the limits, ranges excluding either or both of those included limits are also included in the disclosure.

Embodiments of the present disclosure will employ, unless otherwise indicated, techniques of medicine, organic chemistry, biochemistry, molecular biology, pharmacology, and the like, which are within the skill of the art. Such techniques are explained fully in the literature.

The following examples are put forth so as to provide those of ordinary skill in the art with a complete disclosure and description of how to perform the methods and use the compositions and compounds disclosed and claimed herein. Efforts have been made to ensure accuracy with respect to numbers (*e.g.,* amounts, temperature, *etc*.), but some errors and deviations should be accounted for. Unless indicated otherwise, parts are parts by weight, temperature is in °C, and pressure is at or near atmospheric. Standard temperature and pressure are defined as 20 °C and 1 atmosphere.

Before the embodiments of the present disclosure are described in detail, it is to be understood that, unless otherwise indicated, the present disclosure is not limited to particular materials, reagents, reaction materials, manufacturing processes, dimensions, frequency ranges, applications, or the like, as such can vary. It is also to be understood that the terminology used herein is for purposes of describing particular embodiments only, and is not intended to be limiting. It is also possible in the present disclosure that steps can be executed in different sequence, where this is logically possible. It is also possible that the embodiments of the present disclosure can be applied to additional embodiments involving measurements beyond the examples described herein, which are not intended to be limiting. It is furthermore possible that the embodiments of the present disclosure can be combined or integrated with other measurement techniques beyond the examples described herein, which are not intended to be limiting.

It should be noted that, as used in the specification and the appended claims, the singular forms "a," "an," and "the" include plural referents unless the context clearly dictates otherwise. Thus, for example, reference to "a support" includes a plurality of supports. In this specification and in the claims that follow, reference will be made to a number of terms that shall be defined to have the following meanings unless a contrary intention is apparent.

Prior to describing the various embodiments, the following definitions are provided and should be used unless otherwise indicated.

### Definitions

The term "complement-dependent cytotoxicity" (CDC) as used herein refers to an effector function of IgG and IgM antibodies. When they are bound to surface antigen on target cell (e.g. bacterial or viral infected cell, or a foreign cell such as that of a transplanted organ), the classical complement pathway is triggered by bonding protein C1q to these antibodies, resulting in formation of a membrane attack complex (MAC) and target cell lysis. Complement system is efficiently activated by human IgG1, IgG3 and IgM antibodies, weakly by IgG2 antibodies and it is not activated by IgG4 antibodies.

The term "CD46 complement regulatory protein" (also known as CD46, cluster of differentiation 46, Membrane Cofactor Protein) as used herein refers to a protein encoded by the *CD46* gene and which is CD46 is an inhibitory complement receptor. The gene is found in a cluster with other genes encoding structural components of the complement system. At least fourteen different transcript variants encoding fourteen different isoforms have been found for this gene. The protein encoded by this gene is a type I membrane protein and is a regulatory part of the complement system. The encoded protein has cofactor activity for inactivation (through cleavage) of complement components C3b and C4b by serum factor I, which protects the host cell from damage by complement.

One advantageous target complement inhibitor homologous to CD46 is porcine CD46-like polypeptide having an amino acid sequence according to SEQ ID NO: 23 and which is encoded by a nucleotide sequence having at least 90% sequence identity with the nucleotidde sequence SEQ ID NO: 19.

The term "complement decay-accelerating factor" (also known as CD55 or DAF) as used herein refers to a protein encoded by the *CD55* gene. DAF regulates the complement system on the cell surface. It recognizes C4b and C3b fragments that are created during activation of C4 (classical or lectin pathway) or C3 (alternative pathway). Interaction of DAF with cell-associated C4b of the classical and lectin pathways interferes with the conversion of C2 to C2b, thereby preventing formation of the C4b2b C3-convertase, and interaction of DAF with C3b of the alternative pathway interferes with the conversion of factor B to Bb by factor D, thereby preventing formation of the C3bBb C3 convertase of the alternative pathway. Thus, by limiting the amplification convertases of the complement cascade, DAF indirectly blocks the formation of the membrane attack complex. This glycoprotein is broadly distributed among hematopoietic and non-hematopoietic cells.

The term "CD59 glycoprotein" (also known as MAC-inhibitory protein (MAC-IP), membrane inhibitor of reactive lysis (MIRL), or protectin, is a protein that in humans is encoded by the *CD59* gene. It belongs to the LY6/uPAR/alpha-neurotoxin protein family. CD59 attaches to host cells via a glycophosphatidylinositol (GPI) anchor. When complement activation leads to deposition of C5b678 on host cells, CD59 can prevent C9 from polymerizing and forming the complement membrane attack complex. It may also signal the cell to perform active measures such as endocytosis of the CD59-CD9 complex.

The term "knockout" refers to a transgenic non-human mammal or cell wherein a given gene has been altered, removed or disrupted.

The terms "non-naturally occurring" or "engineered" as used herein are interchangeable and indicate the involvement of the hand of man. The terms, when referring to nucleic acid molecules or polypeptides mean that the nucleic acid molecule or the polypeptide is at least substantially free from at least one other component with which they are naturally associated in nature and as found in nature.

The term "complementarity" as used herein refers to the ability of a nucleic acid to form hydrogen bonds) with another nucleic acid sequence by either traditional Watson-Crick or other non-traditional types. A percent complementarily indicates the percentage of residues in a nucleic acid molecule which can form hydrogen bonds (e.g., Watson-Crick base pairing) with a second nucleic acid sequence (e.g., 5, 6, 7, 8, 9, 10 out of 10 being 50%, 60%, 70%, 80%, 90%, and 100% complementary). "Perfectly complementary" means that all the contiguous residues of a nucleic acid sequence will hydrogen bond with the same number of contiguous residues in a second nucleic acid sequence. "Substantially complementary" as used herein refers to a degree of complementarity that is at least 60%, 65%, 70%, 75%, 80%, 85%. 90%, 95%. 97%, 98%, 99%, or 100% over a region of 8, 9, 10, 11, 12, 13, 14, 15, 16, 17, 18, 19, 20, 21, 22, 23, 24, 25, 30, 35, 40, 45, 50, or more nucleotides, or refers to two nucleic acids that hybridize under stringent conditions.

The term "stringent conditions" for hybridization as used herein refers to conditions under which a nucleic acid having complementarity to a target sequence predominantly hybridizes with the target sequence, and substantially does not hybridize to non-target sequences. Stringent conditions are generally sequence-dependent, and vary depending on a number of factors. In general, the longer the sequence, the higher the temperature at which the sequence specifically hybridizes to its target sequence. Non-limiting examples of stringent conditions are described in detail in Tijssen (1993), Laboratory Techniques In Biochemistry And Molecular Biology-Hybridization With Nucleic Acid Probes Part 1, Second Chapter "Overview of principles of hybridization and the strategy of nucleic acid probe assay", Elsevier, N.Y.

The term "hybridization" as used herein refers to a reaction in which one or more polynucleotides react to form a complex that is stabilized via hydrogen bonding between the bases of the nucleotide residues. The hydrogen bonding may occur by Watson Crick base pairing, Hoogstein binding, or in any other sequence specific manner. The complex may comprise two strands forming a duplex structure, three or more strands forming a multi stranded complex, a single self-hybridizing strand, or any combination of these. A hybridization reaction may constitute a step in a more extensive process, such as the initiation of PCR, or the cleavage of a polynucleotide by an enzyme. A sequence capable of hybridizing with a given sequence is referred to as the "complement" of the given sequence.

The term "expression" as used herein refers to the process by which a polynucleotide is transcribed from a DNA template (such as into and mRNA or other RNA transcript) and/or the process by which a transcribed mRNA is subsequently translated into peptides, polypeptides, or proteins. Transcripts and encoded polypeptides may be collectively referred to as "gene product." If the polynucleotide is derived from genomic DNA, expression may include splicing of the mRNA in a eukaryotic cell.

The term "CRISPR system" as used herein refers collectively to transcripts and other elements involved in the expression of or directing the activity of CRISPR-associated ("Cas") genes, including sequences encoding a Cas gene, a guide sequence (also referred to as a "spacer" in the context of an endogenous CRISPR system), or other sequences and transcripts from a CRISPR locus. In some embodiments, one or more elements of a CRISPR system is derived from a type I, type II, or type III CRISPR system. In some embodiments, one or more elements of a CRISPR system is derived from a particular organism comprising an endogenous CRISPR system, such as *Streptococcus pyogenes.* In general, a CRISPR system is characterized by elements that promote the formation of a CRISPR complex at the site of a target sequence (also referred to as a protospacer in the context of an endogenous CRISPR system).

In the context of formation of a CRISPR complex, "target sequence" refers to a sequence to which a guide sequence is designed to have complementarity, where hybridization between a target sequence and a guide sequence promotes the formation of a CRISPR complex. Full complementarity is not necessarily required, provided there is sufficient complementarity to cause hybridization and promote formation of a CRISPR complex. A target sequence may comprise any polynucleotide, such as DNA or RNA polynucleotides. In some embodiments, a target sequence is located in the nucleus or cytoplasm of a cell. In some embodiments, the target sequence may be within an organelle of a eukaryotic cell, for example, mitochondrion or chloroplast. A sequence or template that may be used for recombination into the targeted locus comprising the target sequences is referred to as an "editing template" or "editing polynucleotide" or "editing sequence". In aspects of the presently disclosed subject matter, an exogenous template polynucleotide may be referred to as an editing template. In an aspect of the presently disclosed subject matter the recombination is homologous recombination.

In some embodiments, a vector comprises one or more insertion sites, such as a restriction endonuclease recognition sequence (also referred to as a "cloning site"). In some embodiments, one or more insertion sites (e.g. about or more than about 1, 2, 3, 4, 5, 6, 7, 8, 9, 10, or more insertion sites) are located upstream and/or downstream of one or more sequence elements of one or more vectors. When multiple different guide sequences are used, a single expression construct may be used to target CRISPR activity to multiple different, corresponding target sequences within a cell. For example, a single vector may comprise about or more than about 1, 2, 3, 4, 5, 6, 7, 8, 9, 10, 15, 20, or more guide sequences. In some embodiments, about or more than about 1, 2, 3, 4, 5, 6, 7, 8, 9, 10, or more such guide-sequence-containing vectors may be provided, and optionally delivered to a cell.

In some embodiments, a vector comprises a regulatory element operably linked to an enzyme-coding sequence encoding a CRISPR enzyme, such as a Cas protein. For example, the amino acid sequence of *S. pyogenes Cas9* protein may be found in the Swissport database under accession number Q99ZW2. In some embodiments, the unmodified CRISPR enzyme has DNA cleavage activity, such as *Cas9.* In some embodiments the CRISPR enzyme is *Cas9,* and may be *Cas9* from *S. pyogenes* or *S. pneumoniae.*

In some embodiments, the CRISPR enzyme directs cleavage of one or both strands at the location of a target sequence, such as within the target sequence and/or within the complement of the target sequence. In some embodiments, the CRISPR enzyme directs cleavage of one or both strands within about 1, 2, 3, 4, 5, 6, 7, 8, 9, 10, 15, 20, 25, 50, 100, 200, 500, or more base pairs from the first or last nucleotide of a target sequence. In some embodiments, a vector encodes a CRISPR enzyme that is mutated to with respect to a corresponding wild-type enzyme such that the mutated CRISPR enzyme lacks the ability to cleave one or both strands of a target polynucleotide containing a target sequence.

In some embodiments, an enzyme coding sequence encoding a CRISPR enzyme is codon optimized for expression in particular cells, such as eukaryotic cells. The eukaryotic cells may be those of or derived from a particular organism, such as a mammal, including but not limited to human, mouse, rat, rabbit, dog, or non-human primate. In general, codon optimization refers to a process of modifying a nucleic acid sequence for enhanced expression in the host cells of interest by replacing at least one codon (e.g. about or more than about 1, 2, 3, 4, 5, 10, 15, 20, 25, 50, or more codons) of the native sequence with codons that are more frequently or most frequently used in the genes of that host cell while maintaining the native amino acid sequence. Various species exhibit particular bias for certain codons of a particular amino acid. Codon bias (differences in codon usage between organisms) often correlates with the efficiency of translation of messenger RNA (mRNA), which is in turn believed to be dependent on, among other things, the properties of the codons being translated and the availability of particular transfer RNA (tRNA) molecules. The predominance of selected tRNAs in a cell is generally a reflection of the codons used most frequently in peptide synthesis. Accordingly, genes can be tailored for optimal gene expression in a given organism based on codon optimization. Codon usage tables are readily available, for example, at the "Codon Usage Database", and these tables can be adapted in a number of ways. Computer algorithms for codon optimizing a particular sequence for expression in a particular host cell are also available. In some embodiments, one or more codons (e.g. 1, 2, 3, 4, 5, 10, 15, 20, 25, 50, or more, or all codons) in a sequence encoding a CRISPR enzyme correspond to the most frequently used codon for a particular amino acid.

In general, a guide sequence is any polynucleotide sequence having sufficient complementarity with a target polynucleotide sequence to hybridize with the target sequence and direct sequence-specific binding of a CRISPR complex to the target sequence. In some embodiments, the degree of complementarity between a guide sequence and its corresponding target sequence, when optimally aligned using a suitable alignment algorithm, is about or more than about 50%, 6/0, 75%, 80, 85%, 90%, 95%, 97.5%/0, 99%, or more. Optimal alignment may be determined with the use of any suitable algorithm for aligning sequences, non-limiting example of which include the Smith-Waterman algorithm, the Needleman-Wunsch algorithm, algorithms based on the Burrows-Wheeler Transform (e.g. the Burrows Wheeler Aligner), ClustalW, Clustal X, BLAT, Novoalign (Novocraft Technologies, ELAND (Illumina, San Diego, Calif.), SOAP (available at soap.genomics.org.cn), and Maq. In some embodiments, a guide sequence is about or more than about 5, 10, 11, 12, 13, 14, 15, 16, 17, 18, 19, 20, 21, 22, 23, 24, 25, 26, 27, 28, 29, 30, 35, 40, 45, 50, 75, or more nucleotides in length. In some embodiments, a guide sequence is less than about 75, 50, 45, 40, 35, 30, 25, 20, 15, 12, or fewer nucleotides in length.

The ability of a guide sequence to direct sequence-specific binding of a CRISPR complex to a target sequence may be assessed by any suitable assay. For example, the components of a CRISPR system sufficient to form a CRISPR complex, including the guide sequence to be tested, may be provided to a host cell having the corresponding target sequence, such as by transfection with vectors encoding the components of the CRISPR sequence, followed by an assessment of preferential cleavage within the target sequence, such as by Surveyor assay as described herein. Similarly, cleavage of a target polynucleotide sequence may be evaluated in a test tube by providing the target sequence, components of a CRISPR complex, including the guide sequence to be tested and a control guide sequence different from the test guide sequence, and comparing binding or rate of cleavage at the target sequence between the test and control guide sequence reactions. Other assays are possible, and will occur to those skilled in the art.

Although the application describes a typical non-human animal (pigs), cells of other animals can similarly be genetically modified. As mentioned above, the pig is desirable in organ transplantation to humans. As used herein, the term "pig" refers to any pig known to the art including, but not limited to, a wild pig, domestic pig, mini pigs, a *Sus scrofa* pig, a *Sus scrofa domesticus* pig, as well as in-bred pigs. Porcine organs, tissues or cells include organs, tissues, devitalized animal tissues, and cells from a pig.

The term "donor organ" as used herein refers to organs, tissue and/or cells from an animal for use as xenografts. The transplant material may be used as temporary or permanent organ replacement for a human subject in need of an organ transplant. Any porcine organ can be used including, but not limited to, the brain, heart, lungs, eye, stomach, pancreas, kidneys, liver, intestines, uterus, bladder, skin, hair, nails, ears, glands, nose, mouth, lips, spleen, gums, teeth, tongue, salivary glands, tonsils, pharynx, esophagus, large intestine, small intestine, small bowel, rectum, anus, thyroid gland, thymus gland, bones, cartilage, tendons, ligaments, suprarenal capsule, skeletal muscles, smooth muscles, blood vessels, blood, spinal cord, trachea, ureters, urethra, hypothalamus, pituitary, pylorus, adrenal glands, ovaries, oviducts, uterus, vagina, mammary glands, testes, seminal vesicles, penis, lymph, lymph nodes and lymph vessels.

The terms "synthetic" and "engineered" as used herein can used interchangeably and refer to a non-naturally occurring material that has been created or modified by the hand of man (e.g., a genetically modified animal or cell having one or predetermined engineered genetic modifications in its genome) or is derived using such material (e.g., a tissue or organ obtained from such genetically modified animal). Cells comprising one or more synthetic or engineered nucleic acids are considered to be engineered or genetically modified cells. As used herein, the term "engineered tissue" refers to aggregates of engineered/genetically modified cells.

Expression of a gene product is decreased when total expression of the gene product is decreased, a gene product of an altered size is produced, or when the gene product exhibits an altered functionality. Thus, if a gene expresses a wild-type amount of product but the product has an altered enzymatic activity, altered size, altered cellular localization pattern, altered receptor-ligand binding or other altered activity, expression of that gene product is considered decreased. Expression may be analyzed by any means known in the art including, but not limited to, RT-PCR, Western blots, Northern blots, microarray analysis, immunoprecipitation, radiological assays, polypeptide purification, spectrophotometric analysis, Coomassie staining of acrylamide gels, ELISAs, 2-D gel electrophoresis, in situ hybridization, chemiluminescence, silver staining, enzymatic assays, ponceau S staining, multiplex RT-PCR, immunohistochemical assays, radioimmunoassay, colorimetric analysis, immunoradiometric assays, positron emission tomography, fluorometric assays, fluorescence activated cell sorter staining of permeabilized cells, radioimmunosorbent assays, real-time PCR, hybridization assays, sandwich immunoassays, flow cytometry, SAGE, differential amplification, or electronic analysis. See, for example, Ausubel et al., eds. (2002) Current Protocols in Molecular Biology, Wiley-Interscience, New York, New York; Coligan et al., (2002) Current Protocols in Protein Science, Wiley-Interscience, New York, New York.

The term "antibody" as used herein refers to an immunoglobulin which specifically binds to and is thereby defined as complementary with a particular spatial and polar organization of another molecule. The antibody can be monoclonal, polyclonal, or a recombinant antibody, and can be prepared by techniques that are well known in the art such as immunization of a host and collection of sera (polyclonal) or by preparing continuous hybrid cell lines and collecting the secreted protein (monoclonal), or by cloning and expressing nucleotide sequences, or mutagenized versions thereof, coding at least for the amino acid sequences required for specific binding of natural antibodies. Antibodies may include a complete immunoglobulin or fragment thereof, which immunoglobulins include the various classes and isotypes, such as IgA, IgD, IgE, IgG1, IgG2a, IgG2b and IgG3, IgM, IgY, etc. Fragments thereof may include Fab, Fv and F(ab')₂, Fab', scFv, and the like. In addition, aggregates, polymers, and conjugates of immunoglobulins or their fragments can be used where appropriate so long as binding affinity for a particular molecule is maintained.

The term "antigen" as used herein refers to any entity that binds to an antibody disposed on an antibody array and induces at least one shared conformational epitope on the antibody. Antigens could be proteins, peptides, antibodies, small molecules, lipid, carbohydrates, nucleic acid, and allergens. An antigen may be in its pure form or in a sample in which the antigen is mixed with other components.

The term "coding sequence" or a sequence which "encodes" a selected polypeptide as used herein refers to a nucleic acid molecule which is transcribed (in the case of DNA) and translated (in the case of mRNA) into a polypeptide *in vivo* when placed under the control of appropriate regulatory sequences (or "control elements"). The boundaries of the coding sequence are determined by a start codon at the 5' (amino) terminus and a translation stop codon at the 3' (carboxy) terminus. A coding sequence can include, but is not limited to, cDNA from viral, prokaryotic or eukaryotic mRNA, genomic DNA sequences from viral or prokaryotic DNA, and even synthetic DNA sequences. A transcription termination sequence may be located 3' to the coding sequence.

The term "primer" as used herein refers to an oligonucleotide complementary to a DNA segment to be amplified or replicated. Typically primers are used in PCR. A primer hybridizes with (or "anneals" to) the template DNA and is used by the polymerase enzyme as the starting point for the replication/amplification process.

The term "expressed" or "expression" as used herein refers to the transcription from a gene to give an RNA nucleic acid molecule at least complementary in part to a region of one of the two nucleic acid strands of the gene. The term "expressed" or "expression" as used herein also refers to the translation from said RNA nucleic acid molecule to give a protein, an amino acid sequence or a portion thereof.

The term "expression vector" as used herein refers to a nucleic acid useful for expressing the DNA encoding the protein used herein and for producing the protein. The expression vector is not limited as long as it expresses the gene encoding the protein in various prokaryotic and/or eukaryotic host cells and produces this protein. When yeast, animal cells, or insect cells are used as hosts, an expression vector preferably comprises, at least, a promoter, an initiation codon, the DNA encoding the protein and a termination codon. It may also comprise the DNA encoding a signal peptide, enhancer sequence, 5'- and 3'-untranslated region of the gene encoding the protein, splicing junctions, polyadenylation site, selectable marker region, and replicon. The expression vector may also contain, if required, a gene for gene amplification (marker) that is usually used.

A promoter/operator region to express the protein in bacteria comprises a promoter, an operator, and a Shine-Dalgarno (SD) sequence (for example, AAGG). For example, when the host is Escherichia, it preferably comprises Trp promoter, lac promoter, recA promoter, lambda.PL promoter, b 1pp promoter, tac promoter, or the like. When the host is a eukaryotic cell such as a mammalian cell, examples thereof are SV40-derived promoter, retrovirus promoter, heat shock promoter, and so on. As a matter of course, the promoter is not limited to the above examples. In addition, using an enhancer is effective for expression. A preferable initiation codon is, for example, a methionine codon (ATG). A commonly used termination codon (for example, TAG, TAA, and TGA) is exemplified as a termination codon. Usually, used natural or synthetic terminators are used as a terminator region. An enhancer sequence, polyadenylation site, and splicing junction that are usually used in the art, such as those derived from SV40, can also be used. A selectable marker usually employed can be used according to the usual method. Examples thereof are resistance genes for antibiotics, such as tetracycline, ampicillin, or kanamycin.

The expression vector used herein can be prepared by continuously and circularly linking at least the above-mentioned promoter, initiation codon, DNA encoding the protein, termination codon, and terminator region to an appropriate replicon. If desired, appropriate DNA fragments (for example, linkers, restriction sites, and so on) can be used by a method such as digestion with a restriction enzyme or ligation with T4 DNA ligase. Transformants can be prepared by introducing the expression vector mentioned above into host cells.

The term "fragment" of a molecule such as a protein or nucleic acid as used herein refers to any portion of the amino acid or nucleotide genetic sequence.

The term "gene" as used herein, refers to a functional protein-, polypeptide-, or peptide-encoding nucleic acid unit.

The term "identity" as used herein refers to a relationship between two or more polypeptide sequences, as determined by comparing the sequences. In the art, "identity" also refers to the degree of sequence relatedness between polypeptides as determined by the match between strings of such sequences. "Identity" and "similarity" can be readily calculated by known methods, including, but not limited to, those described in Computational Molecular Biology, Lesk, A.M., Ed., Oxford University Press, NY, 1988; Biocomputing: Informatics and Genome Projects, Smith, D.W., Ed., Academic Press, NY, 1993; Computer Analysis of Sequence Data, Part I, Griffin, A.M. & and Griffin, H.G., Eds., Humana Press, NJ, 1994; Sequence Analysis in Molecular Biology, von Heinje, G., Academic Press, 1987; Sequence Analysis Primer, Gribskov, M. & Devereux, J., Eds., M Stockton Press, NY, 1991; and Carillo & Lipman (1988) SIAM J. Applied Math., 48: 1073.

Preferred methods to determine identity are designed to give the largest match between the sequences tested. Methods to determine identity and similarity are codified in publicly available computer programs. The percent identity between two sequences can be determined by using analysis software (*i.e.,* Sequence Analysis Software Package of the Genetics Computer Group, Madison, Wis.) that incorporates the Needelman & Wunsch (1970) J. Mol. Biol., 48: 443-453) algorithm (*e.g.,* NBLAST and XBLAST). The default parameters are used to determine the identity for the polypeptides of the present disclosure.

By way of example, a polypeptide sequence may be identical to the reference sequence, that is be 100% identical, or it may include up to a certain integer number of amino acid alterations as compared to the reference sequence such that the percent identity is less than 100%. Such alterations are selected from: at least one amino acid deletion, substitution (including conservative and non-conservative substitution), or insertion, and wherein said alterations may occur at the amino- or carboxy-terminus positions of the reference polypeptide sequence or anywhere between those terminal positions, interspersed either individually among the amino acids in the reference sequence, or in one or more contiguous groups within the reference sequence. The number of amino acid alterations for a given percent identity is determined by multiplying the total number of amino acids in the reference polypeptide by the numerical percent of the respective percent identity (divided by 100) and then subtracting that product from said total number of amino acids in the reference polypeptide.

The term "nucleic acid molecule" as used herein refers to DNA molecules (*e.g.,* cDNA or genomic DNA), RNA molecules (*e.g.,* mRNA), analogs of the DNA or RNA generated using nucleotide analogs, and derivatives, fragments and homologs thereof. The nucleic acid molecule can be single-stranded or double-stranded, but advantageously is double-stranded DNA. An "isolated" nucleic acid molecule is one that is separated from other nucleic acid molecules that are present in the natural source of the nucleic acid. A "nucleoside" refers to a base linked to a sugar. The base may be adenine (A), guanine (G) (or its substitute, inosine (I)), cytosine (C), or thymine (T) (or its substitute, uracil (U)). The sugar may be ribose (the sugar of a natural nucleotide in RNA) or 2-deoxyribose (the sugar of a natural nucleotide in DNA). A "nucleotide" refers to a nucleoside linked to a single phosphate group.

The term "oligonucleotide" as used herein refers to a series of linked nucleotide residues, which oligonucleotide has a sufficient number of nucleotide bases to be used in a PCR reaction. A short oligonucleotide sequence may be based on, or designed from, a genomic or cDNA sequence and is used to amplify, confirm, or reveal the presence of an identical, similar or complementary DNA or RNA in a particular cell or tissue. Oligonucleotides may be chemically synthesized and may be used as primers or probes. Oligonucleotide means any nucleotide of more than 3 bases in length used to facilitate detection or identification of a target nucleic acid, including probes and primers.

The term "transformant" as used herein refers to a recipient cell or cells wherein an expression vector is introduced. As will be understood by those in the art, this functional term includes genomic sequences, cDNA sequences, probes, oligonucleotides or fragments thereof (and combinations thereof), as well as gene products, including those that may have been designed and/or altered by the user. Purified genes, nucleic acids, protein and the like are used to refer to these entities when identified and separated from at least one contaminating nucleic acid or protein with which it is ordinarily associated.

The term "transfection" refers to a process by which agents are introduced into a cell. The list of agents that can be transfected is large and includes, but is not limited to, guide RNA, sense and/or anti-sense sequences, DNA encoding one or more genes and organized into an expression plasmid, proteins, protein fragments, and more. There are multiple methods for transfecting agents into a cell including, but not limited to, electroporation, calcium phosphate-based transfections, DEAE-dextran-based transfections, lipid-based transfections, molecular conjugate-based transfections (*e.g.,* polylysine-DNA conjugates), microinjection and others.

### Discussion

Development of antitumor therapeutic antibodies involves *in vitro* analysis of their effector functions including ability to trigger CDC to kill target cells. The classical approach is to incubate antibodies with target cells and A source of complement (i.e. serum). Then cell death is determined with several approaches:
CDC assays are used to find a suitable donor for organ or bone marrow transplantation, namely donor with matching phenotype of histocompatibility system HLA. After HLA typing is done for patient and donor to determine their HLA phenotypes a crossmatch test is done to exclude that patient produces donor-specific anti-HLA antibodies, which could cause graft rejection.

CDC HLA typing (or serologic typing) uses batch of anti-HLA antibodies from characterized allogeneic antisera or monoclonal antibodies. These antibodies can be incubated one by one with patient's or donor's lymphocytes and a source of complement. The amount of dead cells (and thus positive result) is measured by dead or live cells staining.

Thus, a CDC assay typically involves incubation of a patient's serum with donor's lymphocytes and second incubation after adding rabbit complement. The presence of dead cells (positive test) means that donor isn't suitable for this particular patient. There are modifications available to increase test sensitivity including the extension of minimal incubation time, adding anti-human globulin (AHG), removing unbound antibodies before adding complement, separation of T cell and B cell subset. Besides CDC crossmatch there is flow-cytometric crossmatch available, that is more sensitive and can detect even complement non-activating antibodies. There is still a need, however, to be able to detect very low levels of patient antibodies that have the ability to lyse candidate donor cells, thereby ultimately generating tissue rejection.

The present disclosure encompasses a method of increasing the sensitivity of the complement-dependent cellular cytotoxicity analysis that establishes whether a potential transplant recipient patient expresses donor organ-reactive antibodies that would reduce or prevent acceptance of the donor organ by a recipient. Thus, using a CRISPR Cas9-based procedure expression from at least one gene encoding a complement inhibitor could be inactivated in cells derived from a candidate transplant organ. Such modified cells, because they no longer produce at least one complement inhibitor, when placed in a serum sample from a potential transplant recipient, do not reduce the effective activity of serum complement. Consequently, a lower level of recipient patient serum antibodies becomes effective in inducing lysis of the donor cells. While the methods of the disclosure may be usefully applied to cells derived from a potential donor organ, wherein the cells may be primary cells, cultured cells, or an immortalized cell line generated from isolated donor organ cells and immortalized by techniques known in the art, the methods are also useful when applied to cells such as donor lymphocytes.

One aspect of the disclosure, therefore, encompasses a method of determining whether a candidate donor organ will be the subject of rejection by antibodies of a potential recipient patient. In particular, transplant donor organ-reactive antibodies are detected in a human patient according to the methods defined in the appended claims. In some embodiments, a tissue sample is removed from the candidate organ, and by means of the CRISPR Cas9 gene editing system a gene encoding a complement inhibitor is deleted from the genome of the cells. Suitable targeted complement inhibitors include, but are not limited to CD46-like, CD46, CD55, and CD59. It is contemplated in one embodiment of this method, the necessary time required to generate the genetically engineered cells will not significantly impact the patient.

It is further intended that a genetically modified cells according to the disclosure may be immortalized and cultured or stored by methods well known to those in the art for use in testing multiple serum samples from multiple patients, thus avoiding the necessity of genetically modifying donor-derived cells for each test. This provides a more cost effective and rapidly conducted approach.

The disclosure further provides guide RNA nucleotide sequences suitable for use in inactivating the expression of complement inhibitors by cells from multiple organs of a xenotransplant donor. In one example, not intended to be limiting, the ability to express the complement inhibitor CD46 was eliminated from the genome of renal endothelial cells.

Genome-editing technologies include high-efficiency genome editing through the use of an engineered type II CRISPR/*Cas9* system. The CRISPR constructs, which rely upon the nuclease activity of the *Cas9* protein coupled with a synthetic guide RNA (gRNA), are simple and fast to synthesize and can be multiplexed. However, despite the relative ease of their synthesis, CRISPRs have technological restrictions related to their access to targetable genome space, which is a function of both the properties of *Cas9* itself and the synthesis of its gRNA.

Cleavage by the CRISPR system requires complementary base pairing of the gRNA to a 20-nucleotide DNA sequence and the requisite protospacer-adjacent motif (PAM), a short nucleotide motif found 3' to the target site (Jinek et al., (2012) Science 337: 816-821). One can, theoretically, target any unique N₂₀-PAM sequence in the genome using CRISPR technology. The DNA binding specificity of the PAM sequence, which varies depending upon the species of origin of the specific *Cas9* employed, provides one constraint. Currently, the least restrictive and most commonly used *Cas9* protein is from *S*. *pyogenes,* which recognizes the sequence NGG, and thus, any unique 21-nucleotide sequence in the genome followed by two guanosine nucleotides (N₂₀NGG) can be targeted. Expansion of the available targeting space imposed by the protein component is limited to the discovery and use of novel *Cas9* proteins with altered PAM requirements, or pending the generation of novel *Cas9* variants via mutagenesis or directed evolution.

The second technological constraint of the CRISPR system arises from gRNA expression initiating at a 5' guanosine nucleotide. Use of the type III class of RNA polymerase III promoters has been useful for gRNA expression because these short noncoding transcripts having well-defined ends, and all the necessary elements for transcription, with the exclusion of the 1+ nucleotide, are contained in the upstream promoter region. However, since the commonly used U6 promoter requires a guanosine nucleotide to initiate transcription, use of the U6 promoter has further constrained genomic targeting sites to GN₁₉NGG.

Embodiments of the methods of the invention may utilize gRNAs that target complement inhibitor encoding genes, such as, but not limited, to CD46-like, CD46, CD55, and CD59. In some embodiments, the composition comprises (a) a non-naturally occurring nuclease system (e.g., CRISPR) comprising one or more vectors comprising: i) a promoter (e.g., bidirectional H1 promoter) operably linked to at least one nucleotide sequence encoding a nuclease system guide RNA (gRNA), wherein the gRNA hybridizes with a complement inhibitor-encoding target sequence of a DNA molecule in a cell of the subject, and wherein the DNA molecule encodes one or more gene products expressed in the cell; and ii) a regulatory element operable in a cell operably linked to a nucleotide sequence encoding a genome-targeted nuclease (e.g., *Cas9* protein), wherein components (i) and (ii) are located on the same or different vectors of the system, wherein the gRNA targets and hybridizes with the target sequence and the nuclease cleaves the DNA molecule to alter expression of the one or more gene products.

In some embodiments, the system is packaged into a single adeno-associated virus (AAV) particle or a plasmid. In some embodiments, the adeno-associated virus (AAV) may comprise any of the 51 human adenovirus serotypes (e.g., serotypes 2, 5, or 35). In some embodiments, the system inactivates one or more gene products. In some embodiments, the nuclease system excises at least one gene mutation. In some embodiments, the promoter comprises: a) control elements that provide for transcription in one direction of at least one nucleotide sequence encoding a gRNA; and b) control elements that provide for transcription in the opposite direction of a nucleotide sequence encoding a genome-targeted nuclease. In some embodiments, the *Cas9* protein is codon optimized for expression in the cell. In some embodiments, the promoter is operably linked to at least one, two, three, four, five, six, seven, eight, nine, or ten gRNA.

In some embodiments, the presently disclosed methods of the invention utilize a composition comprising a non-naturally occurring CRISPR system comprising one or more vectors comprising: a) an H1 promoter operably linked to at least one nucleotide sequence encoding a CRISPR system guide RNA (gRNA), wherein the gRNA hybridizes with a target sequence of a DNA molecule in a cell, and wherein the DNA molecule encodes one or more gene products expressed in the cell; and b) a regulatory element operable in a cell operably linked to a nucleotide sequence encoding a *Cas9* protein, wherein components (a) and (b) are located on the same or different vectors of the system, wherein the gRNA targets and hybridizes with the target sequence and the *Cas9* protein cleaves the DNA molecule to alter expression of the one or more gene products.

In some embodiments, the methods of the invention can utilize a composition comprising a non-naturally occurring CRISPR system comprising one or more vectors comprising: a) an H1 promoter operably linked to at least one nucleotide sequence encoding a CRISPR system guide RNA (gRNA), wherein the gRNA hybridizes with a target sequence of a DNA molecule in a eukaryotic cell, and wherein the DNA molecule encodes one or more gene products expressed in the eukaryotic cell; and b) a regulatory element operable in a eukaryotic cell operably linked to a nucleotide sequence encoding a Type-II *Cas9* protein, wherein components (a) and (b) are located on the same or different vectors of the system, whereby the gRNA targets and hybridizes with the target sequence and the *Cas9* protein cleaves the DNA molecule, and whereby expression of the one or more gene products is altered. In one aspect, the target sequence can be a target sequence that starts with any nucleotide, for example, N₂₀NGG. In some embodiments, the target sequence comprises the nucleotide sequence AN₁₉NGG. In some embodiments, the target sequence comprises the nucleotide sequence GN₁₉NGG. In some embodiments, the target sequence comprises the nucleotide sequence CN₁₉NGG. In some embodiments, the target sequence comprises the nucleotide sequence TN₁₉NGG. In some embodiments, the target sequence comprises the nucleotide sequence AN. ₁₉NGG or GN₁₉NGG. In another aspect, the *Cas9* protein is codon optimized for expression in the cell. In another aspect, the *Cas9* protein is codon optimized for expression in the eukaryotic cell. In a further aspect, the eukaryotic cell is a mammalian or human cell. In yet another aspect, the expression of the one or more gene products is decreased.

The invention provides a method of detecting transplant donor organ-reactive antibodies in a patient, the method comprising the steps of: (a) reducing the expression of at least one complement inhibitor by a population of cells derived from a candidate animal transplant donor organ by genetically modifying the population of cells, wherein the transplant donor organ is an organ, tissue or cells from an animal for use as xenografts; (b) contacting the population of genetically-modified cells with a sample of serum isolated from a human patient desiring to receive the transplant donor organ; and (c) detecting lysis of the genetically modified population of cells, wherein lysis indicates if the patient desiring to receive the transplant donor organ expresses donor organ-reactive antibodies, and wherein the lysis is detectable or increased when compared with a population of cells derived from the organ and not genetically modified to have a reduction in at least one expressed complement inhibitor.

In some embodiments, the population of cells can be donor lymphocytes.

In some embodiments, the population of cells can be derived from a kidney.

In some embodiments, the population of cells can be renal endothelial cells.

In some embodiments, the method can further comprise: (i) obtaining a tissue sample from the organ; and (ii) generating a population of genetically-modified organ-derived cells comprising an inactive complement inhibitor encoding gene generated by transfecting the cells with at least one expression vector encoding a guide RNA and CRISPR Cas9, wherein the guide RNA is specific for a complement inhibitor.

In some embodiments, the inactive complement inhibitor encoding gene can be selected from the group consisting of CD46, CD55, and CD59.

In some embodiments, the complement inhibitor can be CD46.

In some embodiments, the guide RNA inactivating an inactive complement inhibitor encoding gene can comprise a nucleotide sequence selected from the group consisting of SEQ ID NO: 1, SEQ ID NO: 3; SEQ ID NO: 5, SEQ ID NO: 7, SEQ ID NO: 9, and SEQ ID NO: 11, and a nucleotide sequence complementary to a nucleotide sequence selected from the group consisting of SEQ ID NO: 2, SEQ ID NO: 4; SEQ ID NO: 6, SEQ ID NO: 8, SEQ ID NO: 10, and SEQ ID NO: 12.

In some embodiments, the complement inhibitor can be CD46-like and the guide RNA for CRISPR Cas9-based inactivation can hybridize under physiological conditions with the sequence of SEQ ID NO: 19, or a complement thereof, and can have a nucleotide sequence of SEQ ID NO: 20.

In some embodiments, the expression vector can be plasmid PX330 or PX458 and the expression plasmid expresses Cas9 when the plasmid is transfected into a mammalian cell.

In some embodiments, the expression vector can comprise a nucleotide sequence having at least 85% similarity to a nucleotide sequence selected from the group consisting of SEQ ID Nos.: 13-18.

The invention also encompasses a method of detecting transplant donor organ-reactive antibodies in a patient, the method comprising the steps of: (a) reducing the expression of at least one complement inhibitor by a population of renal endothelial cells derived from a candidate animal transplant donor kidney by genetically modifying the population of the cells, wherein the genetic modification can be by the steps of: (i) obtaining a tissue sample from the kidney or lymphocytes; and (ii) generating a population of genetically-modified kidney-derived cells comprising an inactive CD46 complement inhibitor-encoding gene generated by transfecting the renal endothelial cells with at least one expression vector encoding a guide RNA and CRISPR Cas9, wherein the guide RNA is specific for the complement inhibitor CD46 and can comprises a nucleotide sequence selected from the group consisting of SEQ ID NO: 1 and SEQ ID NO: 7, and a nucleotide sequence complementary to a nucleotide sequence selected from the group consisting of SEQ ID NO: 2 and SEQ ID NO: 8, and wherein the expression vector can comprise a nucleotide sequence having at least 85% similarity to a nucleotide sequence selected from SEQ ID NO: 13 or 16; (b) contacting the population of genetically-modified renal endothelial cells with a sample of serum isolated from a human patient desiring to receive the transplant donor organ; and (c) detecting lysis of the genetically modified population of renal endothelial cells, wherein lysis indicates if the patient desiring to receive the transplant donor organ expresses donor organ-reactive antibodies, and wherein the lysis is detectable or increased when compared with a population of renal endothelial cells derived from the organ and not genetically modified to have a reduction in at least one expressed complement inhibitor.

Each genetically modified cell may be a mammalian cell, wherein the cell is derived from a candidate animal transplant donor organ tissue, and wherein the cell is genetically modified to have a reduced or no expression of a complement inhibitor.

In some embodiments, the complement inhibitor can be CD46, CD55, or CD59.

In some embodiments, the complement inhibitor can be CD46-like, CD46, CD55, or CD59.

In some embodiments, the complement inhibitor can be CD46-like and can have an amino acid sequence at least 90% similar to the amino acid sequence SEQ ID NO: 23.

In some embodiments, the complement inhibitor can be CD46-like and can be encoded by a nucleotide sequence having at least 90% similarity with the nucleotide sequence of SEQ ID NO: 19.

In some embodiments, the cell can be genetically modified by a deletion by CRISPR Cas9 of all or a fragment of the genome of the cell encoding the complement inhibitor.

In some embodiments, the cell can be a population of cultured cells.

In some embodiments, the donor tissue can be obtained from an organ selected from the group consisting of a kidney, a lung, a heart, muscle, a skin tissue, or lymphocytes.

In some embodiments, the candidate organ is selected from the group consisting of a kidney, a lung, a heart, muscle, and a skin tissue.

In some embodiments, the candidate organ is a kidney.

In some embodiments, the genetically modified mammalian cell is a renal endothelial cell or lymphocyte.

While embodiments of the present disclosure are described in connection with the Examples and the corresponding text and figures, there is no intent to limit the disclosure to the embodiments in these descriptions. The invention is defined by the attached claims.

### EXAMPLES

### Example 1

*Culture of Parent Cell Line:* An Immortalized renal endothelial cell line with GGTA1/B4GaINT2/SLA1 KO was cultured in Rosewell Park Memorial Institute (RPMI) Medium 1640 (gibco,Carlsbad, CA) supplemented with 10% Cosmic calf serum (HyClone, Logan, UT), 100ug/ml endothelial cell-specific growth factor (Corning, Bedford, MA) and 1% Penicillin/Streptomycin (HyClone, Logan, UT) on attachment factor-coated plates (Thermo Fisher, Waltham, MA) at 37 °C and 5% CO₂. Cells were confirmed to be galactose-α1,3-galactose negative by incubation with DyLight 649 *Griffonia simplicifolia* Lectin I, Isolectin B4 (Vector Labs, Burlingame, CA) and SLA class I negative by incubation with SLA class I (Invitrogen, Rockford, IL) and analyzed using at the University of Alabama at Birmingham Comprehensive Flow Cytometry Core on a BD FACS Aria II.

### Example 2

*Generation of gRNAs Expression Vector:* Plasmid pSpCas9(BB)-2A-GFP(PX458) (Addgene plasmid 48138) was used to clone the designed annealed oligos (Table I) to generate guide RNA using the CRISPR associated Cas9 nuclease system. One ug of plasmid pX458 was digested with Bbsl (New England Biolabs, Ipswich, MA) for 30 min at 37 °C. Each pair of phosphorylated oligos were annealed using a Bio-RAD thermal cycler (Applied Biosystems, Foster city, California) starting at 37 °C for 30 min, followed by a step at 95 °C for 5 min and then ramp down to 25 °C at 5 °C/min. Digested pX458 was ligated to the annealed par of oligos for 10 min at room temperature. Ligation reaction was used to transform TOP10 competent cells (Invitrogen, Carlsbad, CA), following the manufacture's protocol. The QlAprep kit (Qiagen, Valencia, CA) was used to isolate plasmid from 8 colonies per treatment. DNA clones were sequenced (Heflin center Genomics Core-DNA, UAB).

### Example 3

*Transfection and Identification of Targeted Cells:* Porcine RECs were transfected by electroporation using the Neon transfection system (Life Technologies, Grand Island, NY, USA) according to the manufacturer's instruction. Concentrations of Cas9-expressing plasmid and cell number remained constant at 2 µg/ 1 x 10⁶ cells per transfection. Cas9expression vectors differed only by the presence or absence of green fluorescent protein (GFP). Cells were cultured for 24 h in antibiotic free culture media. After 48 h, cells transfected with a GFP-expressing vector were sorted on a BD FACS Aria II. A gate was established to retain the brightest cells; this was limited to less than 5% of the total population. Cells with high levels of GFP expression were sorted one cell per well into 96-well plate by the FACS Aria flow cytometer. Cells retained in culture for 14 d until confluent after sorting.

### Example 4

*Genotypic Analysis:* Genomic DNA was isolated from pig cells using the QIAmp.RTM DNA mini kit (Qiagen, Valencia, CA). RNA samples were isolated using the RNeasy Plus Mini Kit (Qiagen, Valencia, CA) following the manufacturer's protocol. RNA quality and quantity were affirmed by NanoDrop spectrophotometer (ND-1000, Wilmington, DE). RNA samples were reverse transcribed using OneStep RT-PCR Kit (Qiagen, Valencia, CA). PCR products were purified and ligated into the pCR4-TOPO TA (Invitrogen, Carlsbad, CA). Transformed bacteria were plated onto Luria-Bertani agar containing 50 µg/ml kanamycin for clone selection. Plasmids were isolated using the QlAprep Spin Miniprep Kit (Qiagen, Valencia, CA, USA). Nucleotide sequences were performed by the Sanger method..

### Example 5

*Phenotypic analysis:* The phenotype-specific mutational efficiency was analyzed by measuring expression of the pig CD46. Cells were stained with Anti-CD46 Antibody (clone 6D8/8, FITC) (Lifespan Biosciences, Seattle, WA) to examine gene expression function. Unlabeled cells were used as a negative control. Flow cytometric data were collected on a BD FACS Aria II, analysis was performed with FlowJo version 10 (Treestar Inc, Ashland, OR).

### Example 6

*Sequence analysis:* Nucleotide sequences were analyzed using Geneious 11.0.4. Overlapping forward and reverse sequence fragments assembled the complete coding sequence of each allele. Multiple sequence alignment was created for each locus. Sequences were confirmed by using NCBI Reference Sequence: NM_213888.1.

### Example 7

*Complement-mediated cytotoxicity:* In a 96-well V-bottom assay plate, 100 µl of serially diluted heat inactivated human serums from thirteen kidney transplant waiting list patients was mixed with a 100 µl aliquot of RECs from parental, Pig CD46KO. Human sera treated with or without the IgM disrupting agent dithiothreitol (DTT) (2.5mM in final) for 30 min at 37 °C. The final concentration of RECs in each well was 1 x 10⁶/ml and serum concentration varied by dilution (100, 50, 25, 12.5, 6.25, 3.125 %). The assay was incubated for 30 min at 4 °C. After incubation, plates were centrifuged for 6 min (400 g), decanted and washed with HBSS; this step was repeated twice. Heat inactivation of human sera prevents the variable presence of complement within stored sera from affecting results. Low-TOX-H Rabbit complement (Cedarlane, Burlington, NC, USA) was diluted 1:15 in HBSS and 140µl was added to each well and incubated for 30 min at 37 °C. After incubation, plates were centrifuged for 6 min (400 g), decanted and washed with HBSS; RECs were labeled with propidium iodide (PI) (2.5mg/ml, Sigma-Aldrich) at room temperature 15 min. Data were collected using a BD Accuri C6 flow cytometer and software (BD Biosciences, Ann Arbor, MI).

### Example 8

*Antibody binding:* 2 x 10⁵ RECs of each group were incubated with 25% heat inactivated human sera from thirteen kidney transplant waiting list patients for 30 min at 4 °C. Samples were then washed three times with Hank's Buffered Salt Solution (HBSS). Human IgG and IgM were detected individually with antihuman secondary antibodies conjugated to Alexa Fluor 488 (Jackson ImmunoResearch Laboratories Inc., West Grove, PA, USA) for 30 min at 4 °C. PBMCs were washed three times in HBSS. Fluorescence detection was performed using an Accuri C6 flow cytometer (Accuri, Ann Arbor, MI, USA). Analysis of the data was performed with FlowJo version 10 (Treestar Inc., Ashland, OR, USA).

### Example 9

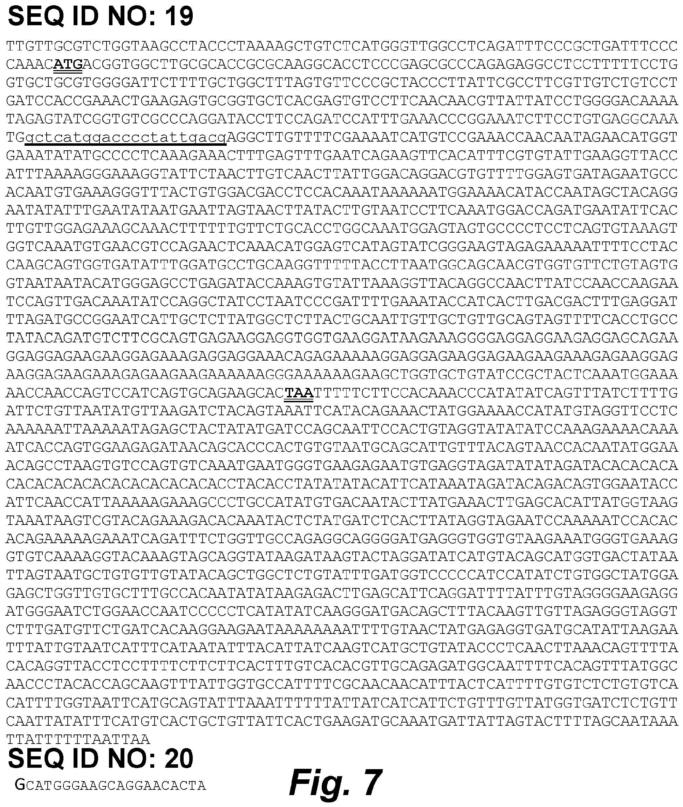

Shown in Fig. 7 is the nucleotide sequence (SEQ ID NO: 19) having the Accession Number XM_003482667.3 and which is an mRNA nucleotide sequence encoding a porcine CD46-like complement inhibitor. An advantageous gRNA sequence is hybridizing to the sequence of SEQ ID NO: 19 is given by SEQ ID NO: 20 (Fig. 7)

### Example 10

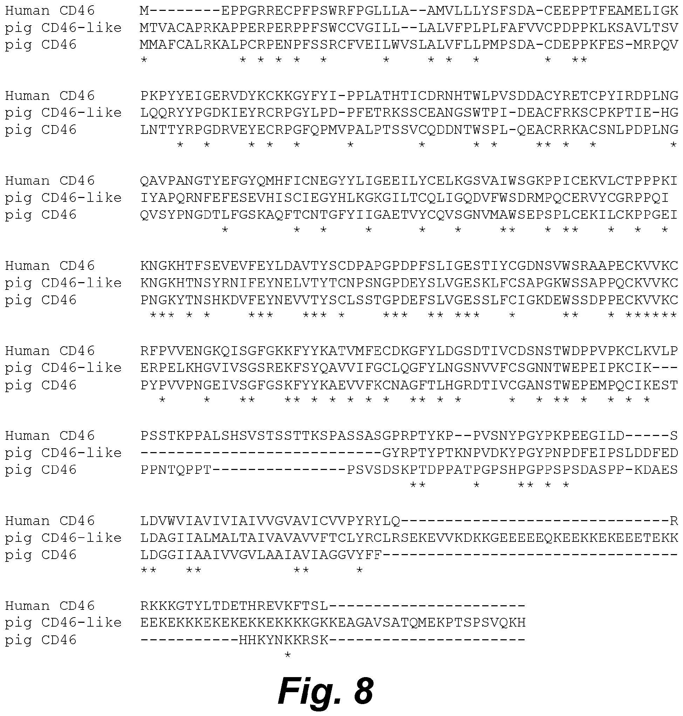

Shown in Fig. 8 is an amino acid sequence alignment showing the similarities between the amino acid sequences human CD46 (SEQ ID NO: 21); porcine CD46 (SEQ ID NO: 22), and porcine CD46-like (SEQ ID NO: 23) complement inhibitors.

## Claims

1. A method of detecting transplant donor organ-reactive antibodies in a human patient, the method comprising the steps of:
(a) reducing the expression of at least one complement inhibitor by a population of cells derived from a candidate animal transplant donor organ by genetically modifying the population of cells, wherein the transplant donor organ is an organ, tissue or cells from an animal for use as xenografts;
(b) contacting the population of genetically-modified cells with a sample of serum isolated from a human patient desiring to receive the transplant donor organ; and
(c) detecting lysis of the genetically modified population of cells, wherein lysis indicates if the patient desiring to receive the transplant donor organ expresses donor organ-reactive antibodies, and wherein the lysis is detectable or increased when compared with a population of cells derived from the organ and not genetically modified to have a reduction in at least one expressed complement inhibitor.

2. The method of claim 1, wherein the animal is a pig.

3. The method of claim 2, wherein the transplant donor organ is a brain, heart, lungs, eye, stomach, pancreas, kidneys, liver, intestines, uterus, bladder, skin, hair, nails, ears, glands, nose, mouth, lips, spleen, gums, teeth, tongue, salivary glands, tonsils, pharynx, esophagus, large intestine, small intestine, small bowel, rectum, anus, thyroid gland, thymus gland, bones, cartilage, tendons, ligaments, suprarenal capsule, skeletal muscles, smooth muscles, blood vessels, blood, spinal cord, trachea, ureters, urethra, hypothalamus, pituitary, pylorus, adrenal glands, ovaries, oviducts, vagina, mammary glands, testes, seminal vesicles, penis, lymph, lymph nodes and lymph vessels.

4. The method of claim 1 or 2, wherein the population of cells are donor lymphocytes.

5. The method of claim 1 or 2, wherein the population of cells are derived from a kidney.

6. The method of claim 5, wherein the population of cells are renal endothelial cells.

7. The method of any of claims 1-3, wherein step (a) further comprises:
(i) obtaining a tissue sample from the organ; and
(ii) generating a population of genetically-modified organ-derived cells comprising an inactive complement inhibitor encoding gene generated by transfecting the cells with at least one expression vector encoding a guide RNA and CRISPR Cas9, wherein the guide RNA is specific for a complement inhibitor.

8. The method of claim 7, wherein the inactive complement inhibitor encoding gene is selected from the group consisting of CD46-like, CD46, CD55, and CD59.

9. The method of claim 7, wherein the complement inhibitor is CD46-like and is encoded by a nucleotide sequence having at least 90% similarity with the nucleotide sequence of SEQ ID NO: 19.

10. The method of claim 7, wherein the complement inhibitor is CD46-like and has an amino acid sequence at least 90% similar to the amino acid sequence SEQ ID NO: 23.

11. The method of claim 7, wherein the guide RNA inactivating an inactive complement inhibitor encoding gene comprises a nucleotide sequence selected from the group consisting of SEQ ID NO: 1, SEQ ID NO: 3; SEQ ID NO: 5, SEQ ID NO: 7, SEQ ID NO: 9, and SEQ ID NO: 11, and a nucleotide sequence complementary to a nucleotide sequence selected from the group consisting of SEQ ID NO: 2, SEQ ID NO: 4; SEQ ID NO: 6, SEQ ID NO: 8, SEQ ID NO: 10, and SEQ ID NO: 12.

12. The method of claim 10, wherein the complement inhibitor is CD46-like and the guide RNA for CRISPR Cas9-based inactivation hybridizing under physiological conditions with a sequence of SEQ ID NO: 19, or a complement thereof, has nucleotide sequence of SEQ ID NO: 20.

13. The method of any one of claims 7-12, wherein CRISPR constructs, which rely upon the nuclease activity of Cas9 protein coupled with a synthetic guide RNA, are multiplexed.

14. The method of claim 7, wherein the expression vector is plasmid PX330 or PX458 and the expression plasmid expresses Cas9 when the plasmid is transfected into a mammalian cell.

15. The method of claim 7, wherein the expression vector comprises a nucleotide sequence having at least 85% similarity to a nucleotide sequence selected from the group consisting of SEQ ID Nos.: 13-18.

## Patentansprüche

1. Ein Verfahren zum Nachweisen von transplantatspenderorganreaktiven Antikörpern bei einem menschlichen Patienten, wobei das Verfahren die folgenden Schritte umfasst:
(a) Reduzieren der Expression von mindestens einem Komplementinhibitor durch eine Population von Zellen, die von einem Kandidatentiertransplantatspenderorgan abstammen, durch genetisches Modifizieren der Population von Zellen, wobei das Transplantatspenderorgan ein Organ, Gewebe oder Zellen aus einem Tier zur Verwendung als Xenotransplantate ist;
(b) In-Kontakt-bringen der Population von genetisch modifizierten Zellen mit einer Probe von Serum, die aus einem menschlichen Patienten isoliert wird, der wünscht, das Transplantatspenderorgan zu empfangen; und
(c) Nachweisen einer Lyse der genetisch modifizierten Population von Zellen, wobei die Lyse angibt, ob der Patient, der wünscht, das Transplantatspenderorgan zu empfangen, spenderorganreaktive Antikörper exprimiert, und wobei die Lyse nachweisbar oder erhöht ist, wenn mit einer Population von Zellen verglichen, die von dem Organ abstammen und nicht genetisch modifiziert sind, um eine Reduzierung in mindestens einem exprimierten Komplementinhibitor aufzuweisen.

2. Verfahren nach Anspruch 1, wobei das Tier ein Schwein ist.

3. Verfahren nach Anspruch 2, wobei das Transplantatspenderorgan ein Gehirn, ein Herz, eine Lunge, ein Auge, ein Magen, ein Pankreas, Nieren, eine Leber, Intestina, einen Uterus, eine Blase, Haut, Haar, Nägel, Ohren, Drüsen, eine Nase, ein Mund, Lippen, eine Milz, Zahnfleisch, Zähne, eine Zunge, Speicheldrüsen, Tonsillen, ein Pharynx, ein Ösophagus, ein großes Intestinum, ein kleines Intestinum, ein Dünndarm, ein Rektum, ein Anus, eine Thyroiddrüse, eine Thymusdrüse, Knochen, Knorpel, Sehnen, Bänder, eine Nebennierenkapsel, Skelettmuskel, glatte Muskel, Blutgefäße, Blut, Rückenmark, eine Trachea, Ureter, eine Urethra, ein Hypothalamus, eine Hypophyse, ein Pylorus, Adrenaldrüsen, Ovarien, Ovidukte, eine Vagina, Brustdrüsen, Testikel, Samenblasen, ein Penis, eine Lymphe, Lymphknoten und Lymphgefäße ist/sind.

4. Verfahren nach Anspruch 1 oder 2, wobei die Population von Zellen Spenderlymphozyten sind.

5. Verfahren nach Anspruch 1 oder 2, wobei die Population von Zellen von einer Niere abstammt.

6. Verfahren nach Anspruch 5, wobei die Population von Zellen renale Endothelzellen sind.

7. Verfahren nach einem der Ansprüche 1-3, wobei Schritt (a) ferner Folgendes umfasst:
(i) Erhalten einer Gewebeprobe von dem Organ; und
(ii) Erzeugen einer Population von genetisch modifizierten organabstammenden Zellen, die ein inaktives Komplementinhibitorcodierungsgen umfassen, das durch Transfizieren der Zellen mit mindestens einem Expressionsvektor erzeugt wird, der eine Guide-RNA und CRISPR-Cas9 codiert, wobei die Guide-RNA für einen Komplementinhibitor spezifisch ist.

8. Verfahren nach Anspruch 7, wobei das inaktive Komplementinhibitorcodierungsgen aus der Gruppe ausgewählt ist, die aus CD46-like, CD46, CD55 und CD59 besteht.

9. Verfahren nach Anspruch 7, wobei der Komplementinhibitor CD46-like ist und durch eine Nukleotidsequenz codiert ist, die mindestens 90% Ähnlichkeit mit der Nukleotidsequenz von SEQ-ID-Nr.: 19 aufweist.

10. Verfahren nach Anspruch 7, wobei der Komplementinhibitor CD46-like ist und eine Aminosäuresequenz aufweist, die mindestens 90 % der Aminosäuresequenz SEQ-ID-Nr.: 23 ähnlich ist.

11. Verfahren nach Anspruch 7, wobei die Guide-RNA-Inaktivierung eines inaktiven Komplementinhibitorcodierungsgens eine Nukleotidsequenz umfasst, die aus der Gruppe ausgewählt ist, die aus SEQ-ID-Nr.: 1, SEQ-ID-Nr.: 3; SEQ-ID-Nr.: 5, SEQ-ID-Nr.: 7, SEQ-ID-Nr.: 9 und SEQ-ID-Nr.: 11 besteht, und eine Nukleotidsequenz komplementär zu einer Nukleotidsequenz umfasst, die aus der Gruppe ausgewählt ist, die aus SEQ-ID-Nr.: 2, SEQ-ID-Nr.: 4; SEQ-ID-Nr.: 6, SEQ-ID-Nr.: 8, SEQ-ID-Nr.: 10 und SEQ-ID-Nr.: 12 besteht.

12. Verfahren nach Anspruch 10, wobei der Komplementinhibitor CD46-like ist und die Guide-RNA für CRISPR-Cas9-basierte Inaktivierung, hybridisierend unter physiologischen Bedingungen mit einer Sequenz von SEQ-ID-Nr.: 19, oder ein Komplement davon, eine Nukleotidsequenz von SEQ-ID-Nr.: 20 aufweist.

13. Verfahren nach einem der Ansprüche 7-12, wobei CRISPR-Konstrukte, die von der Nukleaseaktivität eines mit einer synthetischen Guide-RNA gekoppelten Cas9-Proteins abhängen, multiplexiert werden.

14. Verfahren nach Anspruch 7, wobei der Expressionsvektor Plasmid PX330 oder PX458 ist und das Expressionsplasmid Cas9 exprimiert, wenn das Plasmid in eine Säugerzelle transfiziert wird.

15. Verfahren nach Anspruch 7, wobei der Expressionsvektor eine Nukleotidsequenz umfasst, die mindestens 85 % Ähnlichkeit mit einer Nukleotidsequenz aufweist, die aus der Gruppe ausgewählt ist, die aus SEQ-ID-Nr.: 13-18 besteht.

## Revendications

1. Procédé de détection d'anticorps réactifs à un organe d'un donneur de greffe chez un patient humain, le procédé comprenant les étapes consistant à :
(a) réduire l'expression d'au moins un inhibiteur de complément par une population de cellules dérivées d'un organe de donneur de greffe animal candidat en modifiant génétiquement la population de cellules, l'organe de donneur de greffe étant un organe, un tissu ou des cellules provenant d'un animal et étant destiné à être utilisé comme xénogreffes ;
(b) mettre en contact la population de cellules génétiquement modifiées avec un échantillon de sérum isolé d'un patient humain souhaitant recevoir l'organe de donneur de greffe ; et
(c) détecter une lyse de la population de cellules génétiquement modifiées, la lyse indiquant si le patient souhaitant recevoir l'organe de donneur de greffe exprime des anticorps réagissant à l'organe de donneur, et la lyse étant détectable ou augmentée par rapport à une population de cellules dérivées de l'organe et non génétiquement modifiées pour avoir une réduction dans au moins un inhibiteur de complément exprimé.

2. Procédé selon la revendication 1, dans lequel l'animal est un porc.

3. Procédé selon la revendication 2, dans lequel l'organe de donneur de greffe est un cerveau, un cœur, des poumons, un œil, un estomac, un pancréas, des reins, un foie, des intestins, un utérus, une vessie, une peau, des cheveux, des ongles, des oreilles, des glandes, un nez, une bouche, des lèvres, une rate, des gencives, des dents, une langue, des glandes salivaires, des amygdales, un pharynx, un œsophage, un gros intestin, un intestin grêle, un intestin grêle, un rectum, un anus, une glande thyroïde, un thymus, des os, un cartilage, des tendons, des ligaments, une capsule surrénale, des muscles squelettiques, des muscles lisses, des vaisseaux sanguins, du sang, une moelle épinière, une trachée, des uretères, un urètre, un hypothalamus, une hypophyse, un pylore, des glandes surrénales, des ovaires, des oviductes, un vagin, des glandes mammaires, des testicules, des vésicules séminales, un pénis, une lymphe, des ganglions lymphatiques et des vaisseaux lymphatiques.

4. Procédé selon la revendication 1 ou 2, dans lequel la population de cellules est constituée de lymphocytes de donneur.

5. Procédé selon la revendication 1 ou 2, dans lequel la population de cellules est dérivée d'un rein.

6. Procédé selon la revendication 5, dans lequel la population de cellules est constituée de cellules endothéliales rénales.

7. Procédé selon l'une quelconque des revendications 1 à 3, dans lequel l'étape (a) consiste en outre à :
(i) obtenir un échantillon de tissu à partir de l'organe ; et
(ii) générer une population de cellules dérivées d'organes génétiquement modifiées comprenant un gène codant pour un inhibiteur de complément inactif généré par transfection des cellules avec au moins un vecteur d'expression codant pour un ARN guide et CRISPR Cas9, l'ARN guide étant spécifique d'un inhibiteur de complément.

8. Procédé selon la revendication 7, dans lequel le gène codant pour l'inhibiteur de complément inactif est choisi dans le groupe constitué des protéines de type CD46, CD46, CD55 et CD59.

9. Procédé selon la revendication 7, dans lequel l'inhibiteur de complément est de type CD46 et est codé par une séquence nucléotidique ayant au moins 90 % de similarité avec la séquence nucléotidique SEQ ID NO 19.

10. Procédé selon la revendication 7, dans lequel l'inhibiteur de complément est de type CD46 et comporte une séquence d'acides aminés similaire à au moins 90 % à la séquence d'acides aminés SEQ ID NO 23.

11. Procédé selon la revendication 7, dans lequel l'ARN guide inactivant un gène codant pour un inhibiteur de complément inactif comprend une séquence nucléotidique choisie dans le groupe constitué de SEQ ID NO 1, SEQ ID NO 3, SEQ ID NO 5, SEQ ID NO 7, SEQ ID NO 9 et SEQ ID NO 11, et une séquence nucléotidique complémentaire d'une séquence nucléotidique choisie dans le groupe constitué de SEQ ID NO 2, SEQ ID NO 4, SEQ ID NO 6, SEQ ID NO 8, SEQ ID NO 10 et SEQ ID NO 12.

12. Procédé selon la revendication 10, dans lequel l'inhibiteur de complément est de type CD46 et l'ARN guide pour l'inactivation basée sur CRISPR Cas9, s'hybridant dans des conditions physiologiques avec une séquence SEQ ID NO 19 ou un complément de celle-ci, possède la séquence nucléotidique SEQ ID NO 20.

13. Procédé selon l'une quelconque des revendications 7 à 12, dans lequel les constructions CRISPR, qui reposent sur l'activité nucléasique de la protéine Cas9 couplée à un ARN guide synthétique, sont multiplexées.

14. Procédé selon la revendication 7, dans lequel le vecteur d'expression est le plasmide PX330 ou PX458 et le plasmide d'expression exprime Cas9 lorsque le plasmide est transfecté dans une cellule de mammifère.

15. Procédé selon la revendication 7, dans lequel le vecteur d'expression comprend une séquence nucléotidique ayant au moins 85 % de similarité avec une séquence nucléotidique choisie dans le groupe constitué des SEQ ID NO 13 à 18.
